Europäisches Patentamt

European Patent Office

Office européen des brevets

(11) Numéro de publication : **0 354 100 B1**

# (12) FASCICULE DE BREVET EUROPEEN

(45) Date de publication du fascicule du brevet :
**14.10.92 Bulletin 92/42**

(21) Numéro de dépôt : **89402138.5**

(22) Date de dépôt : **27.07.89**

(51) Int. Cl.$^5$ : **A23J 3/00, C12P 21/06**

(54) **Procédé de traitement de solutions protéiques contenant des pigments tels que, groupements héminiques ou chlorophylles en vue de leur décoloration et produits obtenus.**

Le dossier contient des informations techniques présentées postérieurement au dépôt de la demande et ne figurant pas dans leprésent fascicule.

(30) Priorité : **02.08.88 FR 8810405**

(43) Date de publication de la demande :
**07.02.90 Bulletin 90/06**

(45) Mention de la délivrance du brevet :
**14.10.92 Bulletin 92/42**

(84) Etats contractants désignés :
**AT BE CH DE ES FR GB GR IT LI LU NL SE**

(56) Documents cités :
**EP-A- 0 159 231**
**FR-A- 2 415 968**
**US-A- 4 250 197**

(56) Documents cités :
**FSTA LECTURE - PROCEEDINGS OF THE 6th INTERNATIONAL CONGRESS OF FOOD SCIENCE AND TECHNOLOGY, vol. 9, no. 2, 1983, pages179-180, no. 84-09-s1944, 84057456, Bagsvaerd, DK; F. JACOBSEN et al.: "Decoloration of slaughterhouse blood by an enzyme process"**
**FSTA JOURNAL - JOURNAL OF JAPANSESE SOCIETY OF FOOD SCIENCE AND TECHNO-LOGY NIPPON SHOKUHIN KOGYO GAKKAISHI, vol. 33, no. 6,1986, pages 375-387, Tokyo, JP; T. NAKAMURA et al.: "A study on enzymic hydolysate of red blood cells as food material"**

(73) Titulaire : **CIBEVIAL**
**4-10 avenue du Mont Blanc**
**F-69960 Corbas (FR)**
Titulaire : **IMEDEX**
**58 avenue Leclerc**
**F-69007 Lyon (FR)**

(72) Inventeur : **Coves,, Jacques**
**2 Place de Gordes**
**F-38000 Grenoble (FR)**
Inventeur : **Tayot, Jean-Louis**
**1 rue des Greffières**
**F-69890 La Tour De Salvagny (FR)**

(74) Mandataire : **Bernasconi, Jean et al**
**CABINET LEMOINE ET BERNASCONI 13, Boulevard des Batignolles**
**F-75008 Paris (FR)**

## Description

La présente invention a trait à un procédé de traitement de solutions protéiques contenant des pigments, notamment des groupements héminiques ou des groupements chlorophylles en vue de leur décoloration.

Elle concerne également les produits décolorés obtenus par application dudit procédé.

De nombreuses solutions protéiques de haute valeur biologique, d'origine animale ou végétale, contiennent des pigments dont la couleur n'est pas stable au cours du temps. Elles ne peuvent donc pas être utilisées dans un but alimentaire ou pharmaceutique car elles présentent facilement des défauts de goût ou de coloration.

La valorisation de ces solutions protéiques implique leur décoloration, c'est-à-dire l'obtention, d'une part, de la partie protéique blanche (ou de dérivés de cette partie protéique) et, d'autre part, des pigments.

Si le procédé conforme à l'invention vise plus particulièrement la décoloration de substances issues du sang animal comme l'hémoglobine, il trouve également une application intéressante dans la décoloration des solutions protéiques végétales contenant des chlorophylles.

L'hémoglobine est localisée dans les globules rouges (ou hématies) et se compose de quatre chaînes polypeptidiques (globine) associées chacune à un groupement héminique, constitué d'un noyau tétrapyrrolique et contenant un atome de fer qui peut fixer, de manière réversible, de l'oxygène. Ce groupement héminique est responsable de la couleur rouge du sang.

De façon analogue, un noyau tétrapyrrolique, contenant un atome de magnésium, est responsable de la couleur verte des plantes.

La présence de ces pigments est le plus souvent indésirable dans les produits de transformation de matières premières telles que le sang ou des substances végétales. Ces pigments doivent donc être éliminés afin de récupérer les protéines qui les contiennent et qui sont pratiquement incolores dans la plupart des cas.

Parmi les substances protéiques pigmentées susceptibles d'être valorisées par décoloration, le sang, et plus particulièrement l'hémoglobine, est sans aucun doute le produit présentant la plus haute valeur potentielle et ayant suscité le plus de recherches.

En effet, la France par exemple, produisait en 1980 environ 200.000 tonnes de sang dont 80.000 ont été récupérées pour l'alimentation humaine, celle du bétail ou des animaux de compagnie, la pharmacie,... Les 120. 000 tonnes de sang non récupérées correspondent à 20.000 tonnes de protéines de haute valeur biologique ou technologique qui sont rejetées comme produit de rebut, ce qui représente un énorme gaspillage et une source non négligeable de pollution.

Généralement, le sang d'abattoir est maintenu à l'état liquide, dès la saignée des animaux, par addition d'un anticoagulant classique tel que le citrate de sodium ou les polyphosphates. Ensuite, une simple centrifugation permet de séparer deux fractions d' inégale importance en volume et en composition. La fraction légère et incolore, représentant 60 à 65 % du volume, est nommée plasma et trouve une bonne valorisation en industrie alimentaire (charcuterie, pâtisserie) et en industrie pharmaceutique (production d'albumine essentiellement). Les 35 à 40 % restant constituent la fraction lourde, appelée cruor, et qui contient principalement les hématies. Ce cruor est composé d'environ 30% de protéines dont plus de 90 % sont représentés par l'hémoglobine. C'est justement cette très forte teneur en hémoglobine qui donne au cruor sa coloration rouge intense et nuit à sa valorisation.

Plusieurs brevets ont été pris pour des procédés de décoloration de l'hémoglobine, mais aucun d'eux ne s'est avéré suffisamment rentable ou efficace pour déboucher sur le plan industriel.

Pour séparer le pigment de la protéine (l'hème de la globine), il faut, d'une part, rompre la liaison relativement forte qui les unit et, d'autre part, isoler les deux composés obtenus. Il s'agit là d'une opération délicate car la rupture de la liaison hème-globine nécessite un pH acide (inférieur à 4) et entraîne des modifications structurales telles que la molécule protéique précipite si elle est en solution concentrée. De plus, de part, sa nature hydrophobe, le pigment n'est pas soluble dans l'eau et précipite également.

Des chercheurs ont mis à profit cette propriété hydrophobe de l'hème pour l'extraire à l'aide de solvants organiques acidifiés. Ceci provoque d'un même coup la rupture de la liaison hème-globine, la solubilisation du pigment et la précipitation de la protéine décolorée. On peut, à ce propos, se reporter aux travaux de TYBOR et Coll. -Journal of Food Science 38, 4-6 (1973) où l'on trouve un exemple d'extraction de l'hème par l'acétone acide ou au brevet PCT WO 81/02834 (LINDROOS) qui décrit un exemple d'extraction de l'hème par l'éthanol acide.

Cependant, ces procédés entraînent des coûts d'exploitation prohibitifs à cause des grands volumes de solvants nécessaires pour une décoloration complète des protéines. De plus, outre les problèmes de sécurité, le solvant doit être recyclé et les protéines qui ont précipité sont très difficiles à remettre en solution.

Un autre type de procédé consiste en une acidification à pH inférieur à 3 d'une solution d'hémoglobine diluée d'un facteur 20 à 100, puis en une mise en contact de la solution obtenue avec de la carboxyméthylcellulose (CMC), le pigment libéré par acidification s'adsorbant alors sur la CMC. Il est donné dans le brevet japonais n° 55-008261 (SATO et HAYAKAWA) un exemple de procédé d'adsorption sur colonne. De leur côté, AUTIO et coll., dans Journal of Food Science (1980), 49, 859-862, présentent une version améliorée du même procédé consistant en une adsorption en batch.

Il a été proposé dans le brevet PCT/FR 82/00184 (ESPENAN) une autre méthode de décoloration basée sur l'hydrolyse acide d'une solution très diluée d'hémoglobine. L'hydrolyse est réalisée préférentiellement par l'acide sulfurique, à pH compris entre 2 et 3, et la réaction est accélérée par chauffage de la solution acide à environ 100°C pendant 2 à 3 heures. Un surnageant décoloré est ensuite obtenu par filtration ou centrifugation il contient environ 40 % des protéines initiales. Cette technique est intéressante car très simple, mais elle est coûteuse en énergie nécessaire pour chauffer de grands volumes. De plus, outre un rendement moyen, la majorité des protéines décolorées obtenues sont insolubles à pH neutre car elles précipitent dans une gamme de pH comprise entre 6 et 9 et leur concentration par centrifugation donne un culot marron clair, signe d'une décoloration incomplète.

Une autre catégorie de procédé consiste en une hydrolyse enzymatique d'hémoglobine provenant de cruor hémolysé. Une application est décrite par REGNIER dans la Revue Technique des Vétérinaires et de l'Alimentation (R.T.V.A.), 22, 23-35 (1983). Le cruor est dilué pour obtenir une solution contenant 8 à 19 % de protéines et l'hydrolyse est conduite à une température de 52 à 58°C et à un pH de 8,5 régulé automatiquement pendant les 2 à 4 heures de réaction par un pH stat. L'enzyme utilisée est l'Alcalase NOVO (type subtilisine). La séparation entre les peptides portant l'hème et les peptides décolorés a lieu par centrifugation ou ultrafiltration. La décoloration du jus marron obtenu est poursuivie par addition de charbon actif. Cette opération a également pour but l'adsorption sur le charbon des petits peptides donnant un goût amer et une odeur nauséabonde. L'élimination du charbon par filtration permet d'obtenir une solution jaune contenant de très petits peptides qui ne peuvent être concentrés que par osmose inverse et qui possèdent des propriétés technologiques médiocres.

La demande de brevet FR-A-2.415.968 décrit un procédé de décoloration de solutions sanguines, dans lequel les globules rouges sont hémolysés, partiellement hydrolysés à l'aide d'une enzyme protéolytique, puis l'enzyme est inactivée par chauffage et le caillot qui s'est formé est séparé du surnageant par acidification à pH supérieur à 4, le surnageant pouvant être ensuite traité au charbon actif. L'acidification est de préférence effectuée à l'aide d'acide citrique ou d'acide chlorhydrique.

Il est décrit dans le brevet européen n° 0159231 un autre type d'hydrolyse enzymatique. Les inventeurs hydrolysent une solution à 5 % d'hémoglobine par la pepsine à 37°C pendant 2 à 3 heures. Le pH est ajusté à 2 par l'acide chlorhydrique, puis régulé automatiquement à cette valeur par un pH stat. Ensuite, le surnageant de centrifugation très coloré obtenu est mis en contact sur colonne ou en batch pendant plusieurs heures avec un agent adsorbant choisi parmi la magnésie ou l'alumine. Une nouvelle centrifugation permet d'obtenir un surnageant décoloré ne contenant que 40 à 50 % des protéines initiales.

La présente invention vise à remédier aux inconvénients précités, en proposant un procédé de décoloration de solutions protéiques contenant des groupements pigmentés qui n'utilise ni solvants organiques, ni adsorbants, tout en permettant de récupérer les protéines avec des rendements très élevés et d'obtenir une excellente décoloration.

L'un des buts de la présente invention est de proposer un procédé de décoloration d'une solution d'hémoglobine obtenue par hémolyse de cruor d'abattoir ou d'une solution colorée en vert obtenue par broyage de plantes qui soit d'une mise en oeuvre particulièrement simple et économique.

Un autre but de l'invention est de proposer un procédé de décoloration de solutions protéiques contenant des groupements héminiques, dans lequel il n'est fait appel ni à des solvants organiques, ni à des régulateurs de pH.

Un autre but encore de l'invention est de fournir un produit riche en protéines ou un concentré protéique, pratiquement incolore, et pouvant être utilisé tant dans l'alimentation animale que dans l'alimentation humaine.

Le procédé conforme à l'invention est caractérisé, en ce qu'il consiste à soumettre, dans une première étape, la solution protéique à une hydrolyse enzymatique légère effectuée à un pH et à une température adaptés à l'activité de l'enzyme utilisée puis, dans une seconde étape, à porter la solution partiellement hydrolysée à une température supérieure à 60°C et, de préférence, inférieure ou égale à 80°C, à un pH compris entre 2 et 5 et de préférence 2 à 4, à l'aide d'acide sulfurique.

On peut utiliser avantageusement comme enzyme, lors de l'étape d'hydrolyse, la pepsine à un pH compris entre 2 et 4. De bons résultats sont obtenus avec la pepsine T 1.000, selon Codex 49 (fournies par le Laboratoire Industriel de Biologie) que l'on utilise en proportion généralement comprise entre 0,1 et 0,5 % par rapport à la masse de protéines en solution. Cette enzyme peut être avantageusement solubilisée dans le même acide que celui qui a été utilisé pour ajuster le pH de la solution protéique entre 2 et 4, avant d'être ajoutée à cette solution.

L'hydrolyse enzymatique ménagée à laquelle la solution protéique est soumise lors de la première étape, sert à préparer cette solution à la seconde étape du procédé. Cette seconde étape cumule plusieurs objectifs. Elle permet, tout d'abord, de mieux fluidifier la solution qui s'est épaissie au moment de l'acidification. Elle permet, ensuite, de dénaturer l'enzyme introduite en début de réaction et de provoquer l'agrégation des peptides ou autres substances qui portent les groupements colorés. De plus, l'ensemble du procédé permet, grâce au pH acide et à la température élevée, de réduire ou détruire toute contamination bactérienne.

Lorsque le procédé est appliqué au cruor, celui-ci qui contient généralement de 240 à 300 g d'hémoglobine par litre, est dilué par de l'eau, de préférence d'un facteur de 4 à 6, de manière à obtenir une solution contenant 40 à 75 g d'hémoglobine par litre. On homogénéise la solution par agitation. La dilution provoque la lyse des globules rouges et la libération de l'hémoglobine. La dilution est également nécessaire pour prévenir la coagulation immédiate de l'hémoglobine par acidification.

La solution est amenée à une température voisine de 37°C, et on ajoute l'acide sulfurique, de manière à ajuster le pH entre 2 et 4. Le mélange s'épaissit et sa couleur rouge vire au marron. On ajoute, alors, 50 à 150 mg de pepsine, solubilisée dans le même acide, par litre de solution protéique.

L'hydrolyse enzymatique est effectuée à une température voisine de 37°C pendant 10 à 30 minutes, sans régulation du pH. Celui-ci augmente d'environ 0,5 unité et se stabilise.

La solution est alors, dans une seconde étape, portée à une température supérieure à 60°C et, de préférence, n'excédant pas 80°C durant 15 à 30 minutes, avant d'être centrifugée ou filtrée. On obtient une solution jaune pâle à marron clair, limpide qui contient 80 à 90 % des protéines initiales.

La solution est, ensuite, neutralisée à un pH compris entre 6 et 8 par exemple par de la soude, ce qui provoque la formation d'un précipité foncé, de masse négligeable que l'on sépare par centrifugation. On obtient alors un liquide limpide, légèrement teinté en jaune, contenant 40 à 50 g de protéines par litre.

Les sels générés par la neutralisation peuvent aisément être éliminés par ultrafiltration et la solution est concentrée.

La déshydratation de cette solution concentrée par lyophilisation ou atomisation donne une poudre sensiblement blanche, inodore et insipide. Cette poudre est constituée de peptides d'une masse moléculaire de 1 000 à 10 000 daltons.

Le procédé conforme à l'invention présente par rapport aux procédés antérieurement connus de nombreux avantages :
   – la dilution du cruor est faible et sert essentiellement à hémolyser les hématies pour libérer l'hémoglobine ;
   – aucun agent extérieur devant être recyclé ou éliminé par la suite n'est introduit ; ce procédé ne nécessite ni solvants organiques, ni agents adsorbants ;
   – le temps de réaction est très court et la mise en oeuvre du procédé ne nécessite pas d'appareillage compliqué, et notamment pas de régulateur de pH ;
   – le rendement en protéines ainsi que la décoloration sont excellents ; de plus, les peptides obtenus sont solubles quel que soit le pH ;
   – le procédé est économique car sa mise en oeuvre est extrêmement simple et ne nécessite ni pression, ni températures extrêmes.

Il va être donné, ci-dessous quelques exemples de mise en oeuvre du procédé conforme à l'invention.

Exemple 1 :

Le produit de départ est du cruor de sang bovin préparé à l'abattoir. Sa concentration en hémoglobine est de 260 g/l. 25 ml de cruor sont ajoutés à 125 ml d'eau déminéralisée pour provoquer l'hémolyse des hématies et l'obtention d'une solution à 43,3 g d'hémoglobine par litre.

La solution est amenée à 37°C et le pH ajusté à 3 par quelques gouttes d'acide sulfurique concentré.

25mg de pepsine T1000 selon CODEX 49 en solution dans 1 ml d'$H_2SO_4$, 1 N sont ajoutés et la solution est maintenue à 37°C avec agitation pendant 15 minutes.

Le mélange est ensuite rapidement porté à 80°C et maintenu à cette température en agitant pendant 20 minutes.

10 minutes de centrifugation à 9000 tr/mn permettent de séparer un surnageant de 96 ml qui est neutralisé à pH 7 par NaOH. Après 2 heures de repos à température ambiante, une nouvelle centrifugation de 10 minutes permet d'écarter un léger précipité marron. Le nouveau surnageant obtenu est jaune pâle, parfaitement limpide et possède une concentration en protéines de 44 g/l pour un volume de 95 ml. Le rendement en protéines est donc de 80%.

Exemple 2:

300 ml de cruor bovin dilué 6 fois, contenant 14 g d'hémoglobine, sont portés à 37°C et le pH est ajusté à 2 par de l'acide sulfurique concentré.

40 mg de pepsine T1000, en solution dans 1 ml d'$H_2SO_4$ 0,1 N, sont immédiatement introduits et la réaction se déroule pendant 30 minutes. Au bout de ce laps de temps, le pH s'est stabilisé à 2,6.

La solution est alors portée à 80°C pendant 30 minutes puis centrifugée 15 minutes à 9000 tr/mn.

Les 228 ml de surnageant jaune pâle limpide récupérés contiennent 11,6 g de protéines, ce qui correspond à un rendement de 83 %.

Exemple 3 :

Du cruor bovin est dilué de manière à obtenir une solution à 50 g d'hémoglobine par litre.

150 ml de cette solution sont traités à pH 2 et 37°C pendant 30 minutes par 10 mg de pepsine T1000, puis chauffés à 70°C pendant encore 30 minutes avant d'être centrifugés 10 minutes à 9000 tr/mn.

Le surnageant clair et limpide obtenu est neutralisé à pH 7 et laissé quelque temps à température ambiante. Le léger précipité formé est éliminé par centrifugation. Les 116 ml de produit obtenu contiennent 6,4 g de protéines.

Par diafiltration puis ultrafiltration, la solution est déssalée et concentrée, puis séchée par lyophilisation.

La poudre blanche obtenue est inodore et insipide. Sa masse est de 6,2 g et son analyse permet de tirer les conclusions suivantes

```
- humidité                          3,2 %

- matières minérales résiduelles    1,5 %

- teneur pondérale en peptides      5,9 g
```

L'électrophorèse sur gel de polyacrylamide fait apparaître une bande homogène de masse moléculaire apparente de 3500 à 4000 Daltons.

Exemple 4 :

50 l de cruor bovin frais correspondant à 14 kg d'hémoglobine sont mélangés à 250 l d'eau permutée. 35 g de pepsine T1000 en solution dans 3 l d'$H_2SO_4$ 0,1 N sont ensuite ajoutés au mélange et le pH est ajusté à 2,1 par 27,5 l d'$H_2SO_4$ 1 N.

Le mélange est alors porté à 37°C et maintenu à cette température pendant 30 minutes avec agitation. En fin de réaction, le pH s'est stabilisé à 2,4.

Le mélange est enfin amené à 75°C et maintenu ainsi pendant encore une demi-heure.

Après refroidissement à 44°C, le produit est filtré sur un filtre à plaques et 180 l de filtrat sont récupérés. Le rétentat est lavé sur le filtre par 125 l d'une solution de NaCl à 1,5 g/l, pH 2,5 par $H_2SO_4$ et 150 l de solution de lavage sont récupérés et mélangés au premier filtrat.

Les 330 l de solution ainsi obtenus sont neutralisés à pH 7,2 par 11,15 l de NaOH 2N.

Après une nuit à + 4°C, le mélange est centrifugé et le culot est éliminé. Le surnageant est dessalé par diafiltration, puis concentré par ultrafiltration.

Le concentrat est alors séché par atomisation et 9 kg de poudre pratiquement blanche de dérivés de globine sont récoltés.

Cette poudre peut aisément être remise en solution dans de l'eau, quel que soit le pH de départ.

Exemple 5 :

100 g de luzerne sont finement hachés avec 500 ml d'eau à l'aide d'un hachoir ménager. La soupe verte obtenue est grossièrement filtrée sur une toile de Nylon pour éliminer les plus gros débris.

La suspension est ajustée à pH 2 par quelques gouttes d'acide sulfurique, portée à 37°C et 80 mg de pepsine T1000 en solution dans 2 ml d'$H_2SO_4$ sont ajoutés.

Après 20 minutes de réaction, la température est portée à 80°C et maintenue 30 minutes.

Par centrifugation à 9000 tr/mn pendant 15 minutes, 350 ml de surnageant jaune contenant 5 g de protéines sont obtenus.

## Revendications

1. Procédé de traitement de solutions protéiques contenant des groupements héminiques ou chlorophylles en vue de leur décoloration, caractérisé en ce qu'il consiste à soumettre, dans une première étape, la solution protéique à une hydrolyse enzymatique légère effectuée à un pH et à une température adaptés à l'activité de l'enzyme utilisée, puis, dans une seconde étape, à porter la solution partiellement hydrolysée à une température supérieure à 60°C, à un pH acide, notamment compris entre 2 et 4 ou 5, à l'aide d'acide sulfurique, permettant de fluidifier la solution obtenue et provoquer l'agrégation des groupements colorés.

2. Procédé selon la revendication 1, caractérisé en ce que l'on utilise comme enzyme, lors de l'étape d'hydrolyse, la pepsine à un pH acide compris entre 2 et 4.

3. Procédé selon la revendication 2, caractérisé en ce que la pepsine utilisée est la pepsine T 1000 en proportion généralement comprise entre 0,1 et 0,5 % par rapport à la masse de protéines en solution.

4. Procédé selon l'une des revendications 1 à 3, caractérisé en ce que l'enzyme est solubilisée dans de l'acide sulfurique, avant d'être ajoutée à la solution protéique.

5. Procédé selon l'une des revendications 1 à 4, caractérisé en ce que l'on effectue l'hydrolyse enzymatique pendant de 10 à 30 minutes.

6. Procédé selon la revendication 1, caractérisé en ce qu'on porte la solution à une température supérieure à 60°C pendant de 15 à 30 minutes.

7. Procédé selon la revendication 1 , caractérisé en ce que l'étape de chauffage est suivie de l'élimination du précipité formé, puis de la neutralisation de la solution à un pH compris entre 6 et 8.

8. Procédé selon la revendication 7, caractérisé en ce que la neutralisation s'effectue par de la soude.

9. Procédé selon l'une des revendications 7 et 8, caractérisé en ce que l'on élimine par ultrafiltration les sels générés par la neutralisation et en ce que l'on concentre la solution.

10. Procédé selon l'une des revendications 1 à 9, caractérisé en ce que la solution protéique soumise à l'hydrolyse est une solution d'hémoglobine obtenue par hémolyse de cruor obtenu à partir de sang animal collecté en abattoir.

11. Procédé selon l'une des revendications 1 à 9, caractérisé en ce que la solution protéique est une solution protéique végétale contenant des groupements chlorophylles.

12. Procédé selon l'une des revendications 1 à 10, caractérisé en ce que, lorsqu'il est appliqué à la décoloration du cruor, on dilue, préalablement à l'hydrolyse, le cruor par de l'eau, de préférence d'un facteur de 4 à 6, de manière à obtenir une solution contenant de 40 à 75 g d'hémoglobine par litre et on homogénéise la solution par agitation.

13. Procédé selon la revendication 9, caractérisé en ce que l'on récupère les protéines, sous la forme d'une poudre, par déshydratation de la solution concentrée, par atomisation ou lyophilisation.

14. Produit décoloré susceptible d'être obtenu par application du procédé selon les revendications 1 à 13.

15. Produit décoloré selon la revendication 14, caractérisé en ce qu'il est constitué de peptides d'une masse moléculaire de 1.000 à 10.000 daltons.

## Claims

1. A process for treating proteinic solutions containing haeminic groups or chlorophylls in view of their decolourization, characterized in that it comprises subjecting in a first step the proteinic solution to a slight enzymatic hydrolysis carried out at a pH and temperature which are adapted to the to used enzyme's activity and, in a second step, bringing the partially hydrolyzed solution to a temperature above 60°C, at an acid pH, notably between 2 and 4 or 5, by means of sulfuric acid, in order to fluidify the obtained solution and trigger the agglutination of coloured groups.

2. A process according to claim 1. characterized in that pepsin at an acid pH between 2 and 4 is used as an enzyme during the hydrolysis step.

3. A process according to claim 2, characterized in that the pepsin used is pepsin T 1000 in a proportion generally comprised between 0.1 and 0.5 % in relation to the mass of proteins in solution.

4. A process according to any of claims 1 to 3, characterized in that the enzyme is solubilized in sulfuric acid, before being added to the proteinic solution.

5. A process according to any of claims 1 to 4, characterized in that the enzymatic hydrolysis is carried out during 10 to 30 minutes.

6. A process according to claim 1, characterized in that the solution is brought to a temperature above 60°C during 15 to 30 minutes.

7. A process according to claim 1, characterized in that the heating step is followed by discarding the formed precipitate, then by the neutralization of the solution to a pH between 6 and 8.

8. A process according to claim 1, characterized in that the neutralization is made with soda.

9. A process according to any of claims 7 and 8, characterized in that the salts generated by neutralization are eliminated by ultrafiltration, and that the solution is concentrated.

10. A process according to any of claims 1 to 9, characterized in that the proteinic solution which is subjected to hydrolysis is a haemoglobin solution obtained by haemolysis of cruor obtained from animal blood collected in the slaughterhouse.

11. A process according to any of claims 1 to 9, characterized in that the proteinic solution is a vegetal proteinic solution containing chlorophyll groups.

12. A process according to any of claims 1 to 10, characterized in that when it is applied to the decolourization of cruor, the cruor is before hydrolysis diluted with water, preferably by a factor 4 to 6, so as to obtain a solution containing 40 to 75 g haemoglobin per liter, and that the solution is homogenized by shaking.

13. A process according to claim 9, characterized in that the proteins are collected in powder form by dehydration of the concentrated solution, by atomization or freeze-drying.

14. A decolourized product obtainable by applying a process according to claims 1 to 13.

15. A decolourized product according to claim 14, characterized in that it is made of peptides of molecular weight 1000-10 000 daltons.

**Patentansprüche**

1. Verfahren zur Behandlung und Entfärbung von Proteinlösungen, die Häm- oder Chlorophyllgruppen enthalten, dadurch gekennzeichnet, daß man die Proteinlösung in einem ersten Schritt einer milden enzymatischen Hydrolyse unterwirft, die bei einem pH und einer Temperatur durchgeführt wird, die der Aktivität des verwendeten Enzyms angepaßt sind, und anschließend in einem zweiten Schritt die teilweise hydrolysierte Lösung auf eine Temperatur von höher als 60°C bringt, bei einem mit Hilfe von Schwefelsäure sauren pH, insbesondere zwischen einschließlich 2 und 4 oder 5, was es erlaubt, die erhaltene Lösung zu verflüssigen und die Aggregation der farbigen Gruppen zu bewirken.

2. Verfahren nach Anspruch 1, dadurch gekennzeichnet, daß man als Enzym beim Hydrolyseschritt Pepsin bei einem sauren pH zwischen einschließlich 2 und 4 verwendet.

3. Verfahren nach Anspruch 2, dadurch gekennzeichnet, daß als Pepsin Pepsin T 1000 in einem Prozentsatz von im allgemeinen zwischen einschließlich 0,1 und 0,5%, bezogen auf die Masse des Proteins in der Lösung, verwendet wird.

4. Verfahren nach einem der Ansprüche 1 bis 3, dadurch gekennzeichnet, daß das Enzym vor der Zugabe zu der Proteinlösung in der Schwefelsäure löslich gemacht wird.

5. Verfahren nach einem der Ansprüche 1 bis 4, dadurch gekennzeichnet, daß die enzymatische Hydrolyse 10 bis 30 Minuten lang erfolgt.

6. Verfahren nach Anspruch 1, dadurch gekennzeichnet, daß die Lösung 15 bis 30 Minuten auf eine Temperatur von höher als 60°C gebracht wird.

7. Verfahren nach Anspruch 1, dadurch gekennzeichnet, daß nach dem Schritt des Erwärmens der gebildete Niederschlag entfernt wird und anschließend die Neutralisation der Lösung bis auf einen pH zwischen einschließlich 6 und 8 erfolgt.

8. Verfahren nach Anspruch 7, dadurch gekennzeichnet, daß die Neutralisation mittels Natronlauge erfolgt.

9. Verfahren nach einem der Ansprüche 7 und 8, dadurch gekennzeichnet, daß man die bei der Neutralisation erzeugten Salze durch Ultrafiltration entfernt und die Lösung konzentriert.

10. Verfahren nach einem der Ansprüche 1 bis 9, dadurch gekennzeichnet, daß die der Hydrolyse unterworfene Losung eine Hämoglobinlösung ist, die durch Hämolyse von Bluckuchen gewonnen wurde, der aus im Schlachthaus gesammelten Tierblut erhalten wurde.

11. Verfahren nach einem der Ansprüche 1 bis 9, dadurch gekennzeichnet, daß die der Hydrolyse unterworfene Proteinlösung eine Lösung pflanzlichen Proteins ist, die Chlorophyllgruppen enthält.

12. Verfahren nach einem der Ansprüche 1 bis 10, dadurch gekennzeichnet, daß man bei seiner Anwendung zur Entfärbung des Blutkuchens diesen vor der Hydrolyse mit Wasser, bevorzugt um einem Faktor von 4 bis 6, verdünnt, um eine Lösung mit 40 bis 75g Hämoglobin pro Liter zu erhalten, und die Lösung durch Rühren homogenisiert.

13. Verfahren nach Anspruch 9, dadurch gekennzeichnet, daß man die Proteine durch Dehydratisierung der konzentrierten Lösung, durch Zerstäubung oder durch Lyophilisierung in Form eines Pulvers wiedergewinnt.

14. Entfärbtes Produkt, erhältlich durch Anwendung des Verfahrens nach den Ansprüchen 1 bis 13.

15. Entfärbtes Produkt nach Anspruch 14, dadurch gekennzeichnet, daß es aus Peptiden mit einem Molekulargewicht von 1 000 bis 10 000 Dalton besteht.